# EUROPÄISCHE PATENTSCHRIFT

(11) **EP 0 910 277 B1**
(45) Veröffentlichungstag und Bekanntmachung des Hinweises auf die Patenterteilung: **27.08.2003**
(21) Anmeldenummer: 98919248.9
(22) Anmeldetag: 09.04.1998
(51) Int. Cl.: A61B 1/267, A61B 1/04

(54) **MEDIZINISCHES ENDOSKOP MIT ABGEWINKELTEM GRIFF**
MEDICAL ENDOSCOPE WITH A BENT HANDLE
ENDOSCOPE MEDICAL A MANCHE EN COUDE

(30) Priorität: 14.04.1997 DE 19715507
(43) Veröffentlichungstag der Anmeldung: 28.04.1999
(73) Patentinhaber: Karl Storz GmbH & Co. KG, 78532 Tuttlingen (DE)
(72) Erfinder: RENNER, Klaus, D-78576 Liptingen (DE); IRION, Klaus, M., D-78576 Liptingen (DE); DITTRICH, Horst, D-78194 Immendingen (DE); RUDISCHHAUSER, Jürgen, D-78532 Tuttlingen (DE); KLUMPP, Rudi, D-78532 Tuttlingen (DE)
(74) Vertreter: Weller, Wolfgang, Dr.rer.nat.
(86) Internationale Anmeldenummer: EP9802079
(87) Internationale Veröffentlichungsnummer: WO98046121

(56) Entgegenhaltungen:
- EP-A- 0 501 088
- WO-A-89/11305
- WO-A-95/14425
- US-A- 5 408 992

## Beschreibung

Die Erfindung betrifft ein medizinisches Instrument mit einem tubusartigen Element und mit einem dazu abgewinkelten Griff als eine erste Baugruppe, ferner mit einem Endoskop, das mit einem Kameramodul verbunden ist als eine zweite Baugruppe, mit einer Führung am tubusartigen Element zur Aufnahme eines Schaftes des Endoskopes, sowie mit einem Lichtleiter. Es ist bevorzugt, als Mediastinoskop, als Laryngoskop oder als Divertikuloskop einzusetzen.

Ein derartiges medizinisches Instrument ist aus dem Katalog der Anmelderin "ENDOSKOPE UND INSTRUMENTE FÜR HNO", 5. Ausgabe, 1/1996, Blätter LA2C, LA3D und LA2D, bekannt.

Ein dort gezeigtes Video-Laryngoskop nach Kantor/Berci, Modell II weist als eine erste Baugruppe ein Laryngoskop auf. Das Laryngoskop weist ein tubusartiges Element auf, von dem ein dazu abgewinkelter Griff absteht.

Unter tubusartigem Element im Sinne der vorliegenden Erfindung werden rohrförmige, halbrohrförmige oder auch spatelförmige, sich gegebenenfalls auch verjüngende Elemente verstanden, die in Körperhöhlen, beispielsweise in die Luftröhre, in die Brust oder dergleichen einführbar sind. Der zum tubusartigen Element abgewinkelt abstehende Griff steht unter einem Winkel von 90° oder etwas kleiner ab und dient dazu, das tubusartige Element an Ort und Stelle bringen zu können. Durch das tubusartige Element hindurch oder seitlich in dieses eingeschoben werden Operationsinstrumente eingeführt.

Um die Operationsstelle visuell beobachten zu können, ist am tubusartigen Element eine Führung vorgesehen, die beim vorgenannten Video-Laryngoskop an der Außenseite des tubusartigen Elementes angeordnet ist, in die ein langerstreckter Schaft eines Endoskopes eingeschoben ist. Am proximalen Ende ragt das Endoskop über das tubusartige Element weit hinaus. Vom proximalen Ende des Endoskops steht, meist um 90° gegenüber dem Griff um die Mittellängsachse des tubusartigen Elements verdreht, ein optischer Anschluß vor, an den entweder ein Okular angesetzt ist oder ein Kameramodul einer Videokamera. Das Kameramodul ist über Leitungen mit einer Bildverarbeitungseinheit und mit einem Monitor verbunden.

Vom proximalen Ende des Endoskops steht ein weiterer Anschlußzapfen für einen Lichtleiter vor, der sich in etwa in der Ebene des Griffes, jedoch proximal hinter diesem, seitlich etwa rechtwinklig vom Endoskopschaft weg erstreckt. Über diesen Lichtleiter kann von einer Lichtquelle Licht zu der Operationsstelle geführt werden. Das Licht kann nun über eine separate Leitung am tubusartigen Element oder gleich im Endoskopschaft an das distale Ende herangeführt werden.

Der Zusammenbau des medizinischen Instrumentes weist, wenn man von distal nach proximal längs der Längsachse des tubusartigen Elements sieht, nunmehr zumindest drei Elemente auf, erstens den abgewinkelten Griff, zweitens den dazu verdreht angeordneten, abstehenden Bildleiterstutzen mit daran angebrachtem Kameramodul und den von diesem abführenden Leitungen und drittens den Lichtleiterstutzen, um diesen über eine Lichtleitung mit einer Lichtquelle zu verbinden.

Diese drei unterschiedlichen Bauelemente, die in unterschiedlichen Richtungen abstehen, behindern zum einen die Operationsstelle und insbesondere den Operateur, der ja mit den eigentlichen Operationsinstrumenten durch das tubusartige Element hindurch den operativen Eingriff durchführt.

Zum anderen führen die seitlich abgeführten Elemente dazu, auf das einmal an Ort und Stelle gebrachte Tubuselement ein Drehmoment auszuüben, so daß die Gefahr besteht, daß sich dieses im Laufe der Operation verdreht oder sich, falls eine Person mit den Anschlußleitungen in Berührung tritt, ruckartig verdreht. Daher wurden weitere Maßnahmen dahingehend getroffen, beispielsweise den Griff über weit ausladende Bruststützen an Ort und Stelle zu halten. Dadurch mag möglicherweise ein Drehen verhindert werden können, es wird aber noch ein weiteres zusätzliches, sperriges, stangenförmiges Bauelement notwendig, so daß der Operateur noch weiter behindert ist.

Aus der WO 95 144 25 A ist ein Instrument bekannt, das ein Endoskop mit einem abgewinkelten Griff aufweist. Auf den Schaft des Endoskopes ist zusätzlich ein Tubus aufgeschoben und über eine Stellschraube fixiert.

Vom äußersten patientenfernen Ende des abgewinkelten Griffes des Endoskopes springen zwei Stutzen vor. Ein Stutzen dient zum Anschließen an einen Lichtleiter, ein zweiter Stutzen dient als Koppelstelle für eine Kamera.

Aus der US-A-5 408 992 ist ein langerstrecktes rohrförmiges medizinisches Instrument bekannt, in dessen hohlen Griff ein Kameramodul eingeschoben werden kann.

Es ist daher Aufgabe der vorliegenden Erfindung, hier Abhilfe zu schaffen und ein medizinisches Instrument der eingangs genannten Art zu schaffen, das wesentlich einfacher handhabbar ist und insbesondere wesentlich weniger sperrig baut.

Erfindungsgemäß wird die Aufgabe dadurch gelöst, daß das Endoskop zumindest zwei sich in Richtung des abgewinkelten Griffes hinein erstreckende und in dessen Innenraum aufgenommene Kopplungselemente zum Koppeln mit einem Lichtleiter und zum Koppeln mit dem Kameramodul aufweist, und daß das Kameramodul im Griff aufgenommen ist.

Durch das Vorsehen von den beiden abgewinkelten, sich in den abgewinkelten Griff und in dessen Innenraum hinein erstreckenden Kopplungselementen stehen diese nicht mehr über das tubusartige Element hinaus und bilden also keine von der Außenseite her ersichtliche oder störende sperrigen Anschlüsse. Beide Anschlüsse, also sowohl der Anschluß für den Lichtleiter als auch der Anschluß an das Kameramodul, also der Anschluß, der das Bild leitet, erstrecken sich nun in das Innere des Griffes hinein, sind somit vor versehentlichen Berührungen oder Beeinträchtigungen durch den Griff geschützt. Durch Aufnahme des Kameramodules ebenfalls in dem Griff sind nun all diejenigen Bauelemente, die beim eingangs genannten Stand der Technik igelartig vom tubusartigen Element weggestanden haben, in einer kompakten Bauweise im Inneren des Griffs aufgenommen. Vom äußeren Ende des Griffes stehen dann nur noch die Kabel vor, die den Lichtleiter mit der Lichtquelle und das Kameramodul mit dem Bildverarbeitungssystem verbinden. Der Operateur kann nun von den Bauelementen bzw. den Anschlüssen des Endoskopes und der Kamera unbeeinträchtigt die eigentliche Operation durch das tubusartige Element hindurch durchführen. Das äußere Operationsfeld ist somit nicht durch sperrige Anschlüsse beeinträchtigt.

In einer Ausgestaltung der Erfindung weisen sowohl das tubusartige Element als auch der Griff proximal je eine Öffnung auf, über die das Endoskop und das Kameramodul einführbar sind.

Diese Maßnahme hat nun den Vorteil, daß die Montage der Baugruppen des medizinischen Instrumentes zum Durchführen der Operation und die Demontage nach der Operation, beispielsweise um die einzelnen Teile wieder zu sterilisieren, einfach dadurch durchgeführt werden kann, daß Endoskop und Kameramodul proximal eingeschoben bzw. wieder abgezogen werden.

Dies fördert weiterhin die Vereinfachung der Handhabbarkeit des medizinischen Instruments vor und nach der Operation.

In einer weiteren Ausgestaltung sind die Öffnungen derart ausgebildet, daß ein Zusammenbau aus Endoskop und Kameramodul von proximal nach distal einführbar ist.

Diese Maßnahme hat den erheblichen Vorteil, daß die Bauelemente Endoskop und Kameramodul, bevor sie in das eigentliche medizinische Instrument eingeführt werden, schon zu einer Baueinheit zusammengesetzt werden und funktionsüberprüft werden können. Die komplette Baueinheit braucht dann lediglich noch von proximal her in das tubusartige Element bzw. den Griff eingeschoben werden.

In einer weiteren Ausgestaltung der Erfindung sind Endoskop und Kameramodul einzeln über die Öffnungen einführbar, und diese sind im Instrument miteinander koppelbar.

Diese Maßnahme ist insbesondere dann von Vorteil, wenn sehr lange und extrem dünne Endoskopschäfte eingesetzt werden. In diesem Fall kann z.B. zunächst das Endoskop, noch ohne die Anschlüsse des Lichtleiters oder ohne das Kameramodul, von proximal in das tubusartige Element eingesetzt werden. Anschließend wird über den Griff das Kameramodul mit den Anschlußleitungen eingesetzt und mit dem bereits eingesetzten Endoskop im Griff gekoppelt.

In einer weiteren Ausgestaltung der Erfindung ist das Endoskop mit dem Instrument lösbar verriegelbar, und das Kameramodul ist lösbar mit den Kopplungselementen des Endoskops verbindbar.

Diese Maßnahme hat den Vorteil, daß nun beide zuvor genannten Möglichkeiten, also Einsetzen zunächst nur des Endoskopes oder auch des Zusammenbaus, zu ein und derselben Verriegelung zwischen den eingeschobenen Baueinheiten und dem tubusartigen Element bzw. dem Griff führen, nämlich deswegen, weil diese Verriegelung am Endoskop vorgesehen ist.

Dadurch, daß das Kameramodul lösbar mit dem Kopplungselement des Endoskopes verbindbar ist, können wiederum beide Montageweisen einfach und günstig durchgeführt werden. D.h. es können außerhalb des eigentlichen medizinischen Instrumentes die beiden Bauelemente Endoskop und Kameramodul miteinander gekoppelt werden und dann als Zusammenbau eingesetzt werden, oder es kann zunächst das Endoskop eingesetzt werden und dann das Kameramodul eingesetzt werden und mit dem bereits eingesetzten Endoskop gekoppelt werden.

In einer weiteren Ausgestaltung der Erfindung sind die Innenkontur des Handgriffes und die Außenkontur des Kameramoduls derart aufeinander abgestimmt, daß das mit dem Endoskop gekoppelte Kameramodul nur in Längsrichtung des Griffes abziehbar ist.

Diese Maßnahme ist von besonderem Vorteil, wenn Endoskope mit extrem dünnem und langem Schaft eingesetzt werden. Wird der Zusammenbau aus Endoskop und Kameramodul proximal vom Griff abgezogen und ist ein sehr langer, dünner Endoskopschaft vorhanden, muß die gesamte Baueinheit exakt längs der Längsachse des dünnen Endoskopschaftes geführt vom tubusartigen Element zunächst vollständig abgezogen werden. Da diese Baueinheit irgendwie ergriffen werden muß, erfolgt dies günstigerweise an den vom unteren bzw. äußeren Ende des Griffs vorspringenden Kabeln. Da diese Stelle aber um die Grifflänge vom dünnen Endoskopschaft entfernt ist, besteht die Gefahr, daß beim Abziehen nicht nur eine lineare Abziehbewegung sondern auch eine gewisse Schwenkbewegung durchgeführt wird, mit der Gefahr, daß der Endoskopschaft abbricht oder sich vom verbleibenden Endoskopgehäuse löst. Durch die nunmehr vorgeschlagene Maßnahme ist der Benutzer also gezwungen, das Kameramodul zunächst in Längsrichtung des Griffes zu entkoppeln und abzuziehen, bevor dann das Endoskop vom tubusartigen Element abgezogen werden kann. In diesem Fall kann dann das Endoskop am hinteren Ende des tubusartigen Elementes ergriffen und abgezogen werden, ohne daß die anderen gegebenenfalls relativ schweren Bauelelemente, wie Lichtleiter und Kameramodul, noch daran hängen. Auch dadurch wird erheblich die Handhabungsfreundlichkeit gesteigert.

In einer weiteren Ausgestaltung der Erfindung weist das Kameramodul ein bewegbares Riegelelement auf, das über die Öffnung des Handgriffes von der Außenseite her betätigbar ist.

Diese Maßnahme hat nun den Vorteil, daß der im Griff aufgenommene Zusammenbau aus Endoskop und Kameramodul von der Außenseite durch Betätigen des Riegels gelöst werden kann, ohne daß dazu besondere Aufmerksamkeit oder spezielle Werkzeuge notwendig sind.

In einer weiteren Ausgestaltung der Erfindung ist das Kameramodul mit einer einen Stellring aufweisenden Fokussiervorrichtung versehen, der über die Öffnung des Griffes von der Außenseite her betätigbar ist. Diese Maßnahme hat nun ebenfalls den handhabungsfreundlichen Vorteil, daß nach Einsetzen der Baugruppe Endoskop/Kameramodul fokussiert werden kann, um ein scharfes Bild zu erhalten.

In einer weiteren Ausgestaltung der Erfindung weist der Griff ein etwa U-förmiges Querschnittsprofil auf, wobei die Öffnung des U die proximale Öffnung des Griffes darstellt.

Diese Maßnahme hat den Vorteil, daß ein einfach herstellbarer und auch ergonomisch zu ergreifender Griff geschaffen ist, der die gewünschten Öffnungen aufweist, um das Kameramodul oder gegebenenfalls den Zusammenbau aus Endoskop und Kameramodul in den Griff bzw. das tubusartige Element einzuführen.

In einer weiteren Ausgestaltung der Erfindung weist der Handgriff an dessen äußerem Ende ebenfalls eine Öffnung auf.

Diese Maßnahme hat den Vorteil, daß mit einem solchen Handgriff beide Montagevariationen durchgeführt werden können, also Einschieben des Kameramoduls von proximal oder vom äußeren Ende des Griffes längs dessen Längsachse her.

In einer weiteren Ausgestaltung der Erfindung ist um den Griff ein steriler Überzug vorgesehen.

Diese Maßnahme hat den Vorteil, daß nach Einbringen des Kameramoduls, das ja als solches keine sterile Einheit bildet, der sterile Überzug angelegt wird, somit das medizinische Instrument in den Bereichen, in denen zuvor die nichtsterilen Bauelemente eingesetzt werden, steril umhüllt wird.

Die Erfindung wird nachfolgend anhand einiger ausgewählter Ausführungsbeispiele im Zusammenhang mit den beiliegenden Zeichnungen näher beschrieben und erläutert. Es zeigen:
- Fig. 1: eine Seitenansicht eines ersten Ausführungsbeispiels eines medizinischen Instruments vor der Montage einer ersten Baugruppe aus tubusartigem Element und Griff mit einer zweiten Baugruppe aus Endoskop und Kameramodul,
- Fig. 2: die beiden in Fig. 1 noch getrennten Baugruppen in zusammengesetztem Zustand, wobei außerdem noch um den Griff ein steriler Überzug gelegt ist,
- Fig. 3: eine Situation bei der Demontage des medizinischen Instruments von Fig. 2,
- Fig. 4: ein weiteres Ausführungsbeispiel eines medizinischen Instruments während eines ersten Montageschrittes, bei dem gerade ein Endoskop in das tubusartige Element eingeschoben wird, jedoch ohne Kameramodul,
- Fig. 5: einen Schnitt längs der Linie V-V in Fig. 4, und
- Fig. 6: eine der Darstellung von Fig. 4 vergleichbare Darstellung in einem weiteren Montageschritt, bei dem gerade das Kameramodul in den Griff eingesetzt wird.

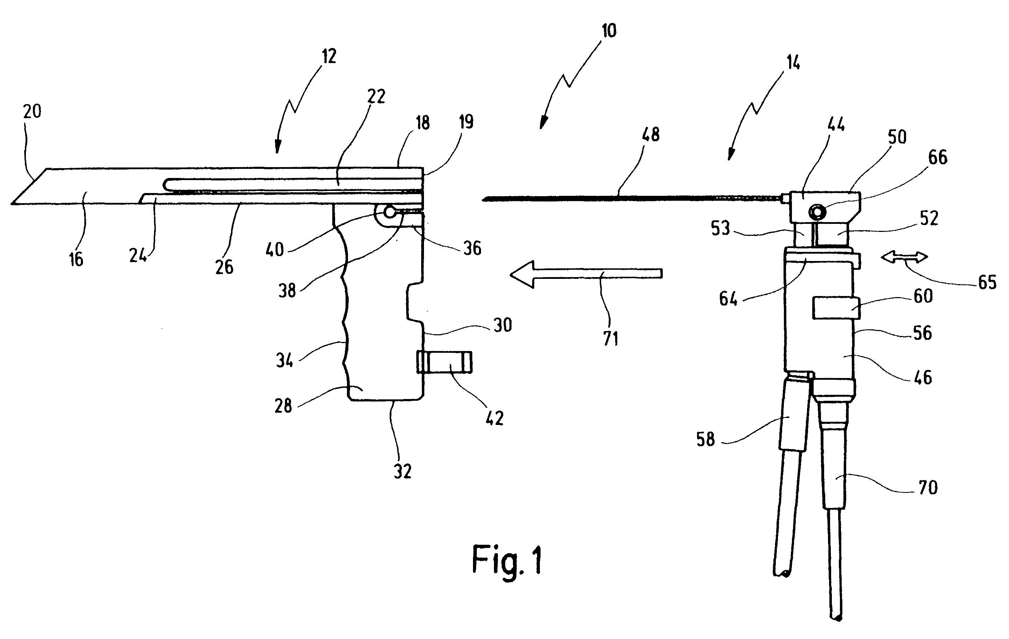

Ein in den Figuren 1 bis 3 gezeigtes erstes Ausführungsbeispiel eines medizinischen Instrumentes ist in seiner Gesamtheit mit der Bezugsziffer 10 versehen.

Wie insbesondere aus der Darstellung von Fig. 1 ersichtlich, besteht das medizinische Instrument 10 aus einer ersten Baugruppe 12 und einer zweiten Baugruppe 14.

Die erste Baugruppe 12 ist ein Mediastinoskop, das ein tubusartiges Element 16 in Form eines zylindrischen Rohres 18 aufweist. Am proximalen Ende ist das Rohr 18 offen, weist somit eine Öffnung 19 auf, am gegenüberliegenden distalen offenen Ende ist dieses abgeschrägt, somit mit einer Spitze 20 versehen.

Das Rohr 18 ist mit einer längs verlaufenden Schlitzöffnung 22 versehen, so daß Instrumente nicht nur proximal über die Öffnung 19 sondern auch seitlich in das Rohr 18 einführbar sind. Im Inneren des Rohres 18 ist eine Führung 24 in Form eines dünnen Rohres 26 vorgesehen, deren Sinn und Zweck später beschrieben ist. Das dünne Rohr 26 ist an der Innenseite des Rohres 18 angelötet.

Am proximalen Ende des Rohres 18 ist, von diesem etwa rechtwinklig abstehend, ein Griff 28 vorgesehen.

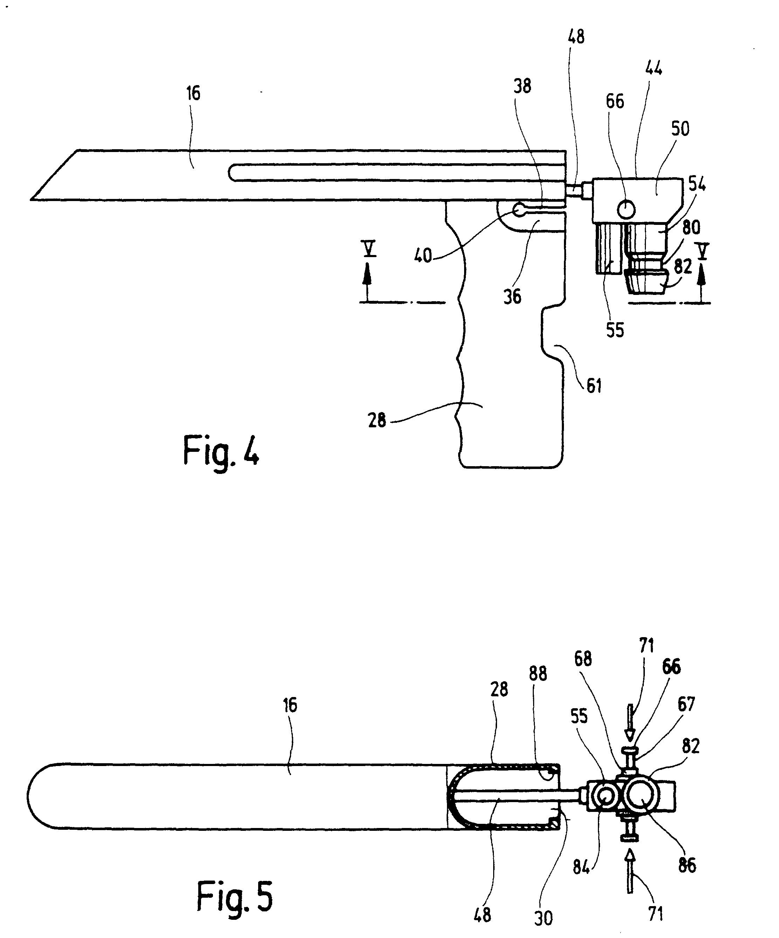

Der Griff 28 weist, wie das später im Zusammenhang mit Fig. 5 beschrieben wird, ein etwa U-förmiges Querschnittsprofil auf, dessen distaler Bereich mit einer Griffmulde 34 zum Anlegen der Finger einer menschlichen Hand versehen ist.

Der Griff 28 ist am proximalen Ende offen, weist somit dort eine durchgehende Öffnung 30 auf, am unteren äußeren Ende ist er ebenfalls offen, weist somit eine Öffnung 32 auf.

Am in der Darstellung von Fig. 1 oberen Ende, also dem Bereich, in dem der Griff 28 mit dem Rohr 28 verbunden ist, ist eine muldenartige Vertiefung 36 vorgesehen, in der sich, parallel zur Mittellängsachse des Rohres 18, ein proximal offener Längsschlitz 38 erstreckt, der an seinem geschlossenen Ende in einen erweiterten Bereich 40 übergeht.

Eine Riegelklappe 42 ist dazu vorgesehen, um die Öffnung 32 teilweise zu verschließen.

Die zweite Baugruppe 14 weist ein Endoskop 44 und ein Kameramodul 46 auf.

Das Endoskop 44 weist einen langen, dünnen, zylindrischen Schaft 48 auf, dessen Länge in etwa der Länge des Rohres 18 des tubusartigen Elementes 16 entspricht, und der in die Führung 24 im Inneren des Rohres 18 eingeschoben werden kann. D.h. der Außendurchmesser des Schaftes 48 entspricht etwa dem lichten Innendurchmesser des dünnen Rohres 26.

Proximal ist der Schaft 48 mit einem Gehäuse 50 verbunden, von dem, unter einem Winkel von etwa 90° zur Schaftachse, zwei Kopplungselemente 52 und 53 vorspringen, deren nähere Ausgestaltung später im Zusammenhang mit den Figuren 4 und 5 beschrieben wird.

Über die Kopplungselemente 52 und 53 ist das Endoskop mit dem Kameramodul 46 lösbar verbunden.

Das Kopplungselement 52 dient zur Kopplung des vom Endoskop 44 erzeugten Bildes mit dem entsprechenden Bildaufnahmesystem des Kameramoduls 46.

Das Kopplungselement 53 dient zur Kopplung der Lichtleiter des Endoskopes 44, die im Schaft 48 aufgenommen sind, mit einer äußeren Lichtquelle.

Das Kameramodul 46 weist ein Gehäuse 56 auf, an dessen den Kopplungselementen 52 und 53 zugewandtem Ende entsprechende weibliche Ausnehmungen vorhanden sind, in die die Kopplungselemente 52 und 53 passend eingreifen können.

Vom gegenüberliegenden Ende des Gehäuses 56 springt ein Lichtleiter 58 vor, der mit einer hier nicht näher bezeichneten Lichtquelle verbunden ist, und der das Licht der Lichtquelle zu dem Kopplungselement 53 führt.

Von demselben Ende führt eine Leitung 70 ab, die das vom Kameramodul 46 erzeugte elektrische Signal einer Bildverarbeitung zuführt.

Zur Erzeugung dieses elektrischen Signales ist im Kameramodul 46 ein hier nicht näher bezeichneter CCD-Sensor (Charge-Coupled-Device-Sensor) vorgesehen, der das vom Endoskop erzeugte Bild in ein elektrisches Signal umsetzt, das über die Leitung 70 abgeführt wird.

Im Kameramodul 46 befindet sich auch noch eine optische Linsenanordnung zum Fokussieren. Dazu ist ein Stellring 60 vorgesehen, um die Fokussiereinrichtung zu verstellen.

An dem den Kopplungselementen 52 und 53 des Endoskopes 44 zugewandten Ende ist am Gehäuse 56 des Kameramoduls 46 ein quer verschiebbares Riegelelement 64 vorgesehen, das, wie das durch einen Doppelpfeil 65 angedeutet ist, hin- und herverschiebbar ist.

Über das verschiebbare Riegelelement 64 kann das Kameramodul 46 lösbar mit dem Endoskop 44 bzw. dessen Kopplungselementen 52 und 53 verbunden bzw. gelöst werden.

Quer zur Längsachse des Schaftes 48 stehen vom Gehäuse 50 des Endoskopes 44 diametral gegenüberstehende Verriegelungsstifte 66 vor, deren nähere Ausgestaltung und Funktion später im Zusammenhang mit den Figuren 4 und 5 beschrieben werden.

Bei der Montage der beiden Baugruppen 12 und 14 zum medizinischen Instrument 10 werden also zunächst Endoskop 44 und Kameramodul 46 miteinander gekoppelt.

Dieser Zusammenbau wird dann, wie das in Fig. 1 durch einen Pfeil 71 dargestellt ist, von proximal nach distal in die erste Baugruppe 12 eingeschoben. Dabei wird der Schaft 48 des Endoskops 44 in die Führung 24 eingeschoben.

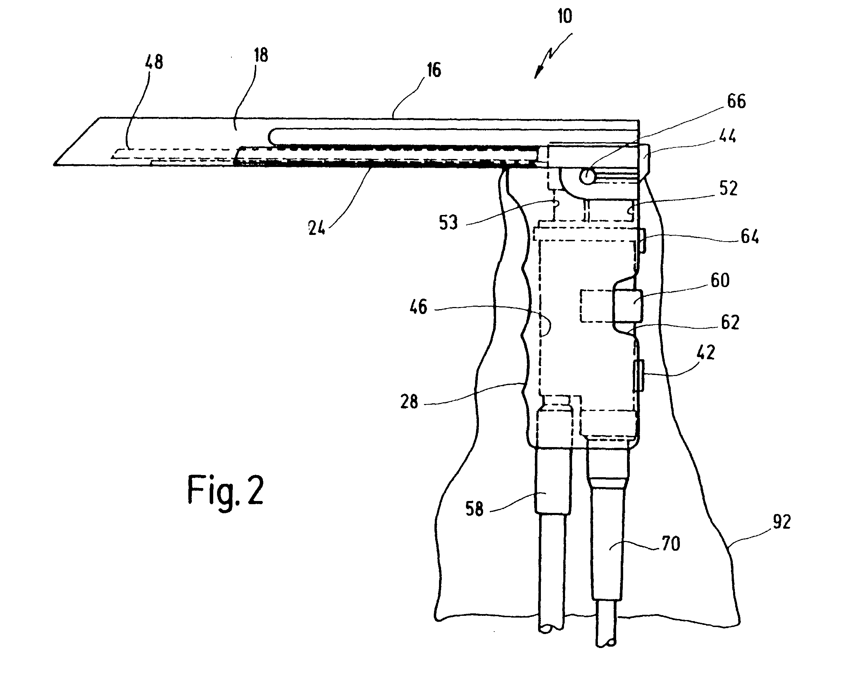

Dieser Verschiebevorgang wird so weit durchgeführt, bis die Verriegelungsstifte 66 in den Längsschlitz 38 am Griff 28 eintreten, dazu sind diese nach innen eingedrückt, und dann den erweiterten Bereich 40 erreicht haben. Dort können die eingedrückten Verriegelungsstifte 66 wieder losgelassen werden und können ausrücken und sperren die so eingeschobene zweite Baugruppe 14 vom Abziehen von der ersten Baugruppe 12. Zur weiteren Sicherung wird nunmehr die Riegelklappe 42 geschlossen, so daß ein Zusammenbau entsteht, wie er in Fig. 2 ersichtlich ist.

Der Stellring 60 des Kameramoduls 46 ist von der Außenseite, von proximal her zugänglich. Um ein Verstellen des Stellringes 60 zu erleichtern, ist in diesem Bereich noch eine zusätzliche Ausnehmung 61 im Griff 28 vorgesehen.

Der Zusammenbau wird anschließend noch mit einem sterilen Überzug 92 in Form einer Folie versehen.

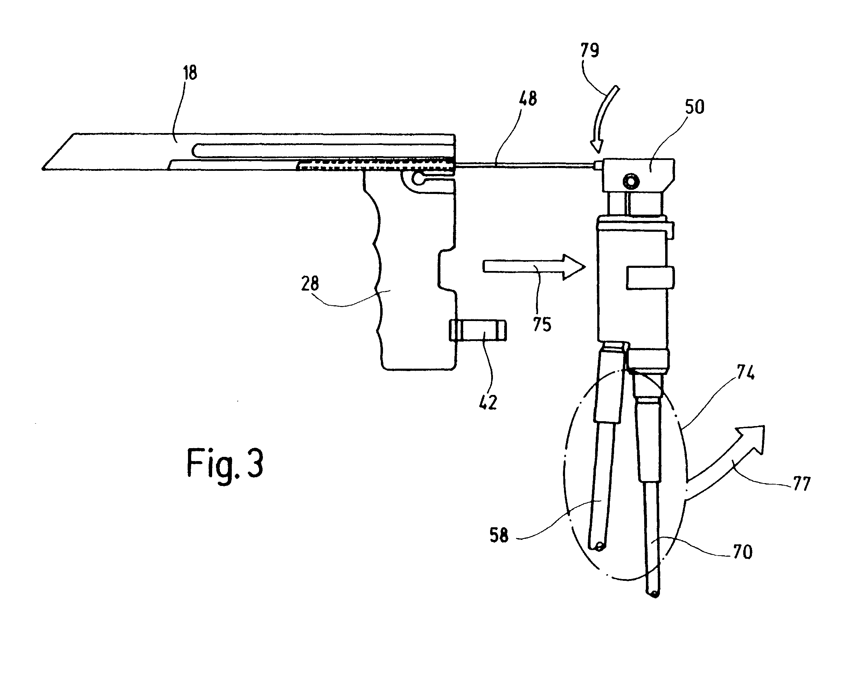

In Fig. 3 ist eine Situation bei der Demontage des Zusammenbaus gezeigt. Es wurde der sterile Überzug 92 abgenommen und die Riegelklappe 42 geöffnet. Da die zweite Baugruppe 14 im Inneren des tubusartigen Elementes 16 bzw. im Inneren des Griffes 28 aufgenommen ist, wird diese zum Abziehen im Bereich der über den Griff 28 hinausstehenden Leitungen 58 und 70 ergriffen, wobei dieser Bereich durch die strichpunktierte Linie 74 umgrenzt ist. Zum Abziehen der zweiten Baugruppe 14 wird diese, wie in Fig. 3 durch einen Pfeil 75 angedeutet ist, abgezogen.

Ist die zweite Baugruppe 14 im Bereich 74 ergriffen, und besteht der Schaft 48 des Endoskopes 44 aus einem extrem langen und dünnen Rohr mit einem Durchmesser im Bereich von einem Millimeter, besteht die Gefahr, daß die Handhabungsperson bei Unachtsamkeiten gleichzeitig beim Abziehen eine Schwenkbewegung durchführt, wie dies in Fig. 3 durch einen Pfeil 77 angedeutet ist. Dadurch wird die Verbindungsstelle zwischen Schaft 48 und Gehäuse 50 des Endoskopes 44 stark belastet, diese Stelle ist in Fig. 3 durch die Spitze des Pfeiles 79 angedeutet, so daß bei unachtsamer Handhabung die Gefahr besteht, daß der Schaft 48 abbricht.

Um diese Gefahr bei Endoskopen mit extrem dünnen Schäften auszuschließen, ist die in den Figuren 4 bis 6 dargestellte Bau- bzw. Montageweise vorgesehen.

Wie aus den Figuren 4 und 5 ersichtlich, wird beim Montieren oder Demontieren ausschließlich das Endoskop 44 eingeschoben bzw. abgezogen. In den Figuren 4 und 5 ist eine Situation dargestellt, bei der der dünne Schaft 48 bereits nahezu über seine volle Länge in die hier nicht näher dargestellte Führung des tubusartigen Elementes 16 eingeschoben ist.

Aus Fig. 5 ist ersichtlich, daß die radial vom Gehäuse 50 des Endoskopes 44 vorspringenden Verriegelungsstifte 66 relativ lang ausgebildet sind und neben einem endseitigen, tellerartigen Endabschnitt einen relativ dünnen Abschnitt 67 und einen inneren, relativ dickeren Abschnitt 68 aufweisen. Der lichte Außendurchmesser des dünnen Abschnittes 67 entspricht etwa der Breite des Längsschlitzes 48, der Außendurchmesser des dickeren Abschnittes 68 entspricht dem Durchmesser des erweiterten Bereiches 40.

In der in den Figuren 4 und 5 dargestellten Position werden nunmehr die federbelasteten Verriegelungsstifte 66 in Richtung Gehäuse 50 gedrückt, wie dies durch Pfeile 71 angedeutet ist.

Die dünnen Abschnitte 67 befinden sich nun in einer solchen Position, daß die Verriegelungsstifte 66 durch den Längsschlitz 38 geschoben werden können.

Wurde das geschlossene Ende des erweiterten Bereiches 40 erreicht, werden die Verriegelungsstifte 66 losgelassen, und diese werden über die hier nicht näher dargestellte Feder nach außen gedrückt, so daß deren innerer, dicker Abschnitt 68 in den erweiterten Bereich 40 des Längsschlitzes 38 einrückt.

Damit die seitlich vorstehenden Verriegelungsstifte 66 später bei der Operation nicht stören, ist dieser Bereich des Griffes 28 mit der Vertiefung 36 versehen.

Aufgrund dieser Vertiefung können aber zur Demontage die Verriegelungsstifte 66 einfach ergriffen und nach innen gedrückt werden, und das Endoskop 44 kann dann wieder abgezogen werden.

Aus der Darstellung von Fig. 4 ist zu erkennen, daß das Kopplungselement 52 als zylindrischer Stutzen 55 ausgebildet ist, der, wie das aus Fig. 5 ersichtlich ist, mit einem Fenster 84 abgeschlossen ist.

Der Stutzen 55 dient als Kopplung für den Lichtleiter 58.

Das sich parallel daneben und im Abstand erstreckende weitere Kopplungselement 52 ist ebenfalls als Stutzen 54 ausgebildet, der mit einer Ringnut 80 versehen ist, an der sich ein Konus 82 anschließt.

Auch der Konus 82 ist, wie dies aus Fig. 5 ersichtlich ist, mit einem Fenster 86 abgeschlossen.

Über den Stutzen 54 wird das vom Endoskop 44 erzeugte Bild abgeleitet.

Dazu wird das im Inneren des Schaftes 48 über ein Linsensystem erzeugte Bild über entsprechende optische Elemente, wie Prismen, seitlich in den Stutzen 54 abgeleitet und dort gegebenenfalls durch ein Zwischenabbildungssystem auf eine Größe gebracht, die dem Fenster 86 entspricht.

Aus der Schnittdarstellung von Fig. 5 ist zu ersehen, daß der Griff 28 am proximalen Ende im Bereich dessen Öffnung 30 mit einer Einengung 88 versehen ist. Diese Einengung 88 existiert allerdings nur im Bereich zwischen der Ausnehmung 61 und dem tubusartigen Element 16.

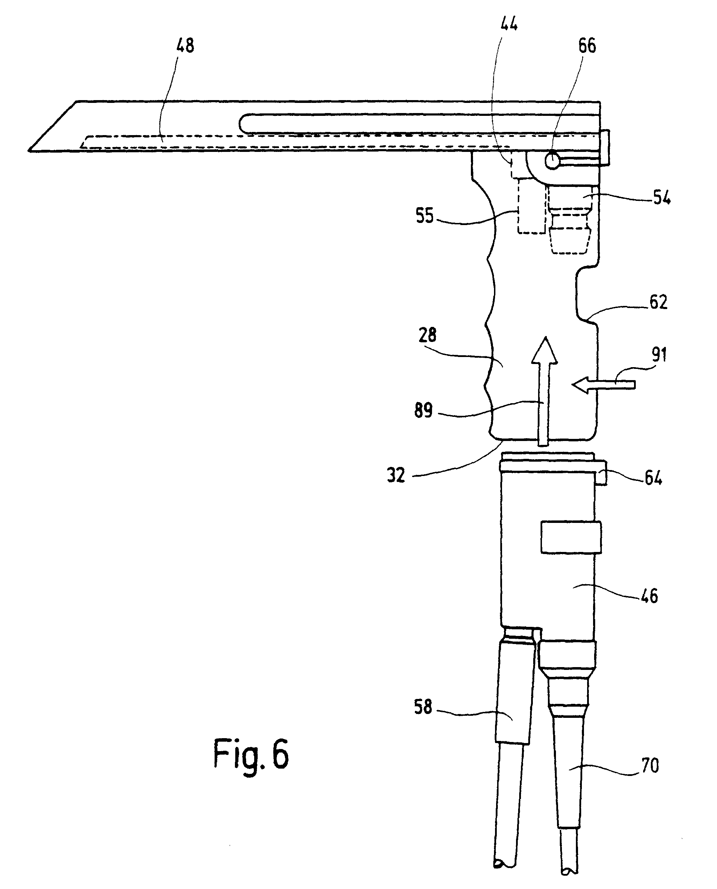

Das Kameramodul 46 kann daher entweder so wie in Fig. 6 dargestellt über die Öffnung 32 oder auch seitlich im Bereich zwischen der Ausnehmung 61 und dem unteren äußeren Ende des Griffes 28 in diesen eingeschoben werden, wie dies durch Pfeile 89 bzw. 91 dargestellt ist.

Anschließend wird das Kameramodul 46 so weit längs des Pfeiles 89 verschoben, bis dieses auf die Stutzen 54 bzw. 55 trifft.

Der Konus 82 des Stutzens 54 fährt dann in eine entsprechende Öffnung ein, schiebt das federbelastete Riegelelement 64 seitlich, bis dieses in die Ringnut 80 einschnappt. In diesem Zustand ist dann das Endoskop 44 mit dem Kameramodul 46 verriegelt, wie es der Darstellung von Fig. 2 entspricht.

Die Einengungen 88 im Bereich des Griffes zwischen der Ausnehmung 61 und dem tubusartigen Element 16 verhindern ein proximales Abziehen dieses Zusammenbaus.

Dazu muß zunächst das Riegelelement 64 gedrückt werden, so daß dieses aus der Ringnut 80 ausrückt, wonach dann das Kameramodul 46 entgegen der Richtung des Pfeiles 89 von den Stutzen 54 bzw. 55 abgezogen werden kann und dann entweder weiter in dieser Richtung oder seitlich entgegengesetzt zur Richtung des Pfeiles 91 vom Griff 28 abgenommen werden kann.

Anschließend wird das Endoskop 44 abgenommen, indem die Verriegelungsstifte 66 eingedrückt und das Endoskop abgezogen werden. Dieser Vorgang ist ohne die Gefahr der Erzeugung eines Drehmomentes, wie es im Zusammenhang mit Fig. 3 beschrieben wurde, zu bewerkstelligen, so daß nicht die Gefahr besteht, daß bei dünnen Endoskopschäften der Schaft abgebrochen oder abgerissen wird.

## Patentansprüche

1. Medizinisches Instrument mit einem tubusartigen Element (16), und mit einem dazu abgewinkelten Griff (28) als eine erste Baugruppe (12), ferner mit einem Endoskop (44), das mit einem Kameramodul (46) verbunden ist als eine zweite Baugruppe (14), mit einer Führung (24) am tubusartigen Element (16) zur Aufnahme eines Schaftes (48) des Endoskopes (44), sowie mit einem Lichtleiter (58), **dadurch gekennzeichnet, daß** das Endoskop (44) zumindest zwei sich in Richtung des abgewinkelten Griffes (28) hinein erstreckende und in dessen Innenraum aufgenommene Kopplungselemente (52, 53) zum Koppeln mit einem Lichtleiter (58) und zum Koppeln mit dem Kameramodul (46) aufweist, und daß das Kameramodul (46) im Griff (28) aufgenommen ist.

2. Medizinisches Instrument nach Anspruch 1, **dadurch gekennzeichnet, daß** das tubusartige Element (16) und der Griff (28) proximal je eine Öffnung (19, 30) aufweisen, über die das Endoskop (44) und das Kameramodul (46) einführbar sind.

3. Medizinisches Instrument nach Anspruch 2, **dadurch gekennzeichnet, daß** die Öffnungen (19, 30) derart ausgebildet sind, daß ein Zusammenbau aus Endoskop (44) und Kameramodul (46) von proximal nach distal einführbar ist.

4. Medizinisches Instrument nach Anspruch 2, **dadurch gekennzeichnet, daß** Endoskop (44) und Kameramodul (46) einzeln über die Öffnungen (19 bzw. 30) einführbar sind, und im Instrument (10) miteinander koppelbar sind.

5. Medizinisches Instrument nach einem der Ansprüche 1 bis 4, **dadurch gekennzeichnet, daß** das Endoskop (44) mit dem Instrument (10) lösbar verriegelbar ist, und daß das Kameramodul (46) lösbar mit den Kopplungselementen (52, 53) des Endoskopes (44) verbindbar ist.

6. Medizinisches Instrument nach Anspruch 4, **dadurch gekennzeichnet, daß** die Innenkontur des Griffes (28) und die Außenkontur des Kameramoduls (46) derart aufeinander abgestimmt sind, daß das mit dem Endoskop (44) gekoppelte Kameramodul (46) nur in Längsrichtung des Griffes (28) abziehbar ist.

7. Medizinisches Instrument nach einem der Ansprüche 2 bis 6, **dadurch gekennzeichnet, daß** das Kameramodul (46) ein bewegbares Riegelelement (64) aufweist, das über die Öffnung (30) des Handgriffes (28) von der Außenseite her betätigbar ist.

8. Medizinisches Instrument nach einem der Ansprüche 2 bis 7, **dadurch gekennzeichnet, daß** das Kameramodul (46) eine einen Stellring (60) aufweisende Fokussiereinrichtung aufweist, wobei der Stellring (60) über die Öffnung (30) des Griffes (28) von der Außenseite her betätigbar ist.

9. Medizinisches Instrument nach einem der Ansprüche 2 bis 8, **dadurch gekennzeichnet, daß** der Griff (28) ein etwa U-förmiges Querschnittsprofil aufweist, wobei die Öffnung des U die proximale Öffnung (30) des Griffes (28) darstellt.

10. Medizinisches Instrument nach einem der Ansprüche 2 bis 9, **dadurch gekennzeichnet, daß** der Griff (28) an dessen äußerem Ende ebenfalls eine Öffnung (32) aufweist.

11. Medizinisches Instrument nach einem der Ansprüche 1 bis 10, **dadurch gekennzeichnet, daß** um den Griff (28) ein steriler Überzug (92) vorgesehen ist.

## Claims

1. Medical instrument having a tube-like element (16) and a handle (28) bent off thereof, providing a first assembly (12), further an endoscope (44) connected with a camera module (46) providing a second assembly (14), a guide (24) provided on the tube-like element (16) for receiving a shaft (48) of the endoscope (44), and a light guide (58), **characterized in that** said endoscope (44) comprises at least two coupling elements (52, 53) extending in the direction and into said bent-off handle (28) and being received in its inside, for being coupled to a light guide (58), and for being coupled to said camera module (46), and wherein said camera module (46) is disposed inside said handle (28).

2. The medical instrument of Claim 1, **characterized in that** said tube-like element (16) and said handle (28) are provided with one opening (19, 30) each at their proximal ends through which said endoscope (44) and said camera module (46) can be introduced.

3. The medical instrument of Claim 2, **characterized in that** the openings (19, 30) are configured in such a way that an assembly consisting of said endoscope (44) and said camera module (46) can be introduced from the proximal toward the distal end.

4. The medical instrument of Claim 2, **characterized in that** said endoscope (44) and said camera module (46) can be introduced via said openings (19 and/or 30) separately, and can be coupled inside the instrument.

5. The medical instrument of anyone of Claims 1 to 4, **characterized in that** said endoscope (44) can be detachably locked on said instrument (10), and the camera module (46) can be detachably connected with said coupling elements (52, 53) of said endoscope (44).

6. The medical instrument of Claim 4, **characterized in that** the inner contour of said handle (28) and the outer contour of said camera module (46) are mutually adapted in such a way that the camera module (46), coupled with endoscope (44), can be pulled off only in the longitudinal direction of said handle (28).

7. The medical instrument of anyone of Claims 2 to 6, **characterized in that** said camera module (46) comprises a movable locking element (64) that can be operated from the outside through said opening (30) of said handle (28).

8. The medical instrument of anyone of Claims 2 to 7, **characterized in that** said camera module (46) is provided with a focusing device comprising a set-collar (60) that can be actuated from the outside through said opening (30) of said handle (28).

9. The medical instrument of anyone of Claims 2 to 8, **characterized in that** said handle (28) exhibits a substantially U-shaped cross-sectional profile, with the opening of the U representing said proximal opening (30) of said handle (28).

10. The medical instrument of anyone of Claims 2 to 9, **characterized in that** said handle (28) is provided with another opening (32) at its outer end.

11. The medical instrument of anyone of Claims 1 to 10, **characterized in that** said handle (28) is provided with a sterile cover (92).

## Revendications

1. Instrument médical avec un élément (16) de type tube et avec une poignée (28) coudée par rapport à celui-ci en tant que premier ensemble (12), avec en outre un endoscope (44) qui est relié à un module de caméra (46) en tant que deuxième ensemble (14), avec un guide (24) sur l'élément (16) de type tube pour recevoir une tige (48) de l'endoscope (44), ainsi qu'avec un guide de lumière (58), **caractérisé en ce que** l'endoscope (44) comporte au moins deux éléments de couplage (52, 53) s'étendant à l'intérieur en direction de la poignée (28) coudée et reçus dans son volume intérieur pour le couplage avec un guide de lumière (58) et pour le couplage avec le module de caméra (46), et **en ce que** le module de caméra (46) est logé dans la poignée (28).

2. Instrument médical selon la revendication 1, **caractérisé en ce que** l'élément (16) de type tube et la poignée (28) présentent chacun côté proximal une ouverture (19, 30) par laquelle l'endoscope (14) et le module de caméra (46) peuvent être introduits.

3. Instrument médical selon la revendication 2, **caractérisé en ce que** les ouvertures (19, 30) sont réalisées de manière qu'un ensemble constitué de l'endoscope (44) et du module de caméra (46) puisse être introduit du côté proximal vers le côté distal.

4. Instrument médical selon la revendication 2, **caractérisé en ce que** l'endoscope (44) et le module de caméra (46) peuvent être introduits séparément à travers les ouvertures (19 ou 30) et être couplés entre eux dans l'instrument (10).

5. Instrument médical selon l'une des revendications 1 à 4, **caractérisé en ce que** l'endoscope (44) peut être verrouillé de manière séparable avec l'instrument (10) et **en ce que** le module de caméra (46) peut être relié de manière séparable aux éléments de couplage (52, 53) de l'endoscope (54).

6. Instrument médical selon la revendication 4, **caractérisé en ce que** le contour intérieur de la poignée (28) et le contour extérieur du module de caméra (46) sont adaptés l'un à l'autre de manière que le module de caméra (46) accouplé à l'endoscope (44) ne puisse être retiré que dans le sens de la longueur de la poignée (28).

7. Instrument médical selon l'une des revendications 2 à 6, **caractérisé en ce que** le module de caméra (46) comporte un élément de verrouillage (64) déplaçable qui peut être actionné depuis le côté extérieur, à travers l'ouverture (30) de la poignée (28).

8. Instrument médical selon l'une des revendications 2 à 7, **caractérisé en ce que** le module de caméra (46) comporte un dispositif de focalisation présentant une bague de réglage (60), la bague de réglage (60) pouvant être actionnée depuis le côté extérieur, à travers l'ouverture (30) de la poignée (28).

9. Instrument médical selon l'une des revendications 2 à 8, **caractérisé en ce que** la poignée (28) présente un profil de section transversale approximativement en U, l'ouverture du U constituant l'ouverture proximale (30) de la poignée (28).

10. Instrument médical selon l'une des revendications 2 à 9, **caractérisé en ce que** la poignée (28) présente également une ouverture (32) à son extrémité extérieure.

11. Instrument médical selon l'une des revendications 1 à 10, **caractérisé en ce qu'**un revêtement (92) stérile est prévu autour de la poignée (28).
